# EUROPEAN PATENT SPECIFICATION

(11) **EP 1 604 690 B1**
(45) Date of publication and mention of the grant of the patent: **05.03.2014**
(21) Application number: 04013513.9
(22) Date of filing: 08.06.2004
(51) Int. Cl.: A61L 9/012, A61L 9/04, A61L 9/12, A01M 1/20, A01N 25/10, A61K 9/10

(54) **Polymeric hot melt adhesive compositions for sustained release of volatile materials**
Polymere, heissklebende Zusammensetzung zur verzögerten Freisetzung von flüchtigen Substanzen
Composition polymère thermofusible pour la liberation prolongée de matières volatiles

(43) Date of publication of application: 14.12.2005
(73) Proprietor: THE PROCTER & GAMBLE COMPANY, Cincinnati, OH 45202 (US)
(72) Inventor: Corzani, Italo, 66100 Cieti (IT); MacBeath, Calum, 66023 Francavilla al Mare(Chieti) (IT); Mariani, Manuel, 65015 Montesilvano (IT)
(74) Representative: Yorquez Ramirez, Maria Isabel

(56) References cited:
- DE-A- 10 237 066
- US-A- 3 876 762
- US-A- 5 698 166
- DATABASE WPI Section Ch, Week 199702 Derwent Publications Ltd., London, GB; Class A17, AN 1997-017519 XP002293455 & JP 08 283484 A (BRIDGESTONE CORP) 29 October 1996 (1996-10-29)
- DATABASE WPI Section Ch, Week 198537 Derwent Publications Ltd., London, GB; Class A17, AN 1985-226871 XP002293456 & JP 60 147443 A (MITSUI TOATSU CHEM INC) 3 August 1985 (1985-08-03)
- DATABASE WPI Section Ch, Week 198441 Derwent Publications Ltd., London, GB; Class A17, AN 1984-252973 XP002293457 & JP 59 152151 A (NIPPON OIL KK) 30 August 1984 (1984-08-30)

## Description

### Field of the Invention

The present invention relates to plasticized hot melt adhesive polymeric compositions which are able to incorporate and sustainedly release perfumes based on copolymers of ethylene with at least another monomer comprising at least a heteroatom. The compositions of the present invention can find a variety of applications wherein a prolonged delivery of a volatile material in an environment is desired such as in air freshening devices, deodorants, scented objects, insecticides etc. or when there is a need to create a long lasting perfumed headspace in a package.

### Background of the Invention

Polymeric compositions which are able to absorb and release volatile ingredients are well known in the art, in particular concerning perfume delivery.

GB1558960 from Nagae, describes a perfume emitting PVC film to be used in umbrellas.

US 4618629 from T. Burnett & Co, Inc describes fragrance-emitting polyurethane foams which have a particulate fragrance-carrying resin incorporated in them. The resin can be selected from a list of polymers (polyolefins, polyester, PVC and similar, polyamides, cellulose polymers).

A common use of polymeric compositions for perfume delivery comprises for example air freshening devices. These are typically in the form of aqueous gels usually obtained from crosslinked polysaccharide polymers (starches, alginates or CMC) such as those described in GB2286531 from Kelco, US3969280 from Johnson & Johnson.

While these and other documents claim to provide long lasting delivery of volatile materials, they are still far from being fully satisfactory for a number of reasons.

Firstly, these polymeric compositions can normally incorporate and release a very limited amount of volatile material, in most cases not exceeding 10% of the total weight of the compositions.

Secondly these polymeric compositions are not able to release uniformly different components of the volatile material which have different volatilities. Given, for example, a perfume which can have more than 10 different components, the more volatile components will be released first and after some time only the less volatile notes would be perceptible, therefore the full perfume character would never be perceived by the user. Actually, the above mentioned polymeric compositions are commonly used to deliver simple perfumes, typically consisting of a single volatile substance such as citronellol as they are simply not capable to consistently deliver a more sophisticated perfume as is increasingly desired by the modem perfume industry.

Thirdly pure polymeric materials are difficult to transform and generally require high temperatures to be molded. Therefore whenever a volatile material is introduced in the melt, a large amount of said material is lost due to the high temperature.

The first and second problems have been partially addressed by Firmenich in US 4734278 which describes shaped bodies of block polyether-amide based resins (e.g. Pebax™) that provide sustained release of volatile actives (perfumes, deodorants, insecticides etc). An improvement has been obtained by Atochem that in WO 9726020A1 describes improved fragrant resins made of Pebax™ plus a complex perfume (i.e. more than 5 components). Such resins are able to deliver a complex perfume with a reduced separation of the volatile ingredients over time.

The third problem has been only partially solved by the use of plasticizers which, as it is well known to those skilled in the art, allow the reduction of the processing temperature of polymeric mixtures. This solution has been applied e.g. by Avon in US4552693 which describes transparent fragrance-emitting articles obtained from compositions comprising a thermoplastic polyamide resin, a plasticizer/solvent system comprising a sulfonamide plasticizer, and a fragrance. The advantage of using a plasticizer in these compositions is the possibility of processing said compositions (molding , extruding, filming) at relatively low temperatures, as it is known for so called hot melt compositions.

Further improved compositions have been described in European Patent Application Publication No. 1531169 A (Procter & Gamble Company) which describes a polymeric material which is able to incorporate and sustainedly release volatile materials which are composed by several ingredients of different volatility for a long time and without separation of said ingredients during the release, and is also capable of being easily processed and formed into an article, preferably with hot melt techniques.

Another problem which is relevant when polymeric compositions capable of delivering a volatile material are to be incorporated into a product is their adhesion on substrates when applied e.g. in the molten state. In many cases, in fact, said polymeric compositions need to be applied on a substrate (e.g. the internal surface of a package, or a support surface on an air freshener device) on a high speed process line. In this situation it is clearly desirable not only that the polymeric compositions can be applied as a hot melt but also that said polymeric compositions have sufficient adhesion to remain in the desired point of application without the need for an additional source of adhesion such as an adhesive strip, or an additional hot melt glue to be applied onto the substrate before said polymeric composition in order to fix it on that. Prior art materials have to compromise between capability in delivering different volatile materials and effective adhesion properties in the molten state.

Prior art polymeric compositions are often based on specific pure polymers like polyether-polyamide block copolymers, pure polyamide polymers or pure EVA polymers. As a consequence the choice of the volatile material was limited to those ingredients which were soluble or compatible with that specific polymer.

In US 5,861,128 a composition comprising an EVA polymer and a plasticizer is described. However the plasticizers suggested for use with EVA are only hydrocarbons (e.g. polybutene) and therefore the resulting compositions are able to incorporate only a limited number of perfume materials due to their low polarity.

US 4,515,909 describes resinous compositions for the release of fragrant substances which are based on EVA and also contain up to 10% of the weight of the polymer of a perfume diffusing agent which can also be regarded as a plasticizer, but the low level at which it is used does not provide the composition with adhesion properties, in fact compositions described here are used in molds and are not adhesive.

Therefore there is still a need for a polymeric composition which is able to incorporate and sustainedly release different volatile materials, which is also capable of being easily processed, typically as a hot melt, and which has a good adhesion on most polymeric and cellulosic substrates.

The compositions of the present invention have very good handling of high amounts of different volatile materials (high wt% storage, long sustained delivery times) combined with good adhesive properties which allow easy application where needed for incorporation in a product in an industrial manufacturing process.

### Summary of the Invention

The present invention relates to a polymeric composition according to claim 1.

### Detailed Description of the Invention

It was surprisingly found that a polymeric composition according to claim 1 has the ability of releasing said volatile material for a long time in a sustained manner, i.e. with a constant release rate and for a long period of time. Such compositions can be applied as hot-melt adhesives and also have surprisingly good adhesion on most substrates (plastic films, foams, cardboard and the like).

Another very important benefit provided by the polymeric compositions of the present invention is the possibility to introduce a wide range of volatile materials.

It has been surprisingly found that polymeric compositions according to the present invention, can incorporate and effectively deliver a large number of volatile materials in a broad polarity range, while also having a good adhesion in the molten state on most substrates.

Differently from prior art, the compositions of the present invention are much more flexible in terms of the compositions of the volatile material which can be incorporated and then delivered, since the formulator can choose the copolymer among all copolymers of ethylene with at least another monomer comprising at least a heteroatom, additionally the plasticizer can be selected among a wide range of suitable materials of different polarity and behavior. Also, a number of additives can be optionally introduced into the formulation as explained in detail below. Such a formulation flexibility for the plasticized polymeric matrix (copolymer, plasticizer, optionally other polymers or additives) allows the tuning of its polarity characteristics very precisely. This makes it possible to maximize the compatibility with any volatile material which could be introduced in the plasticized polymeric matrix thus obtaining a polymeric composition according to the present invention. Without being bound to any theory, it is believed that a certain polarity match between the plasticized polymeric matrix and the volatile material is required to provide good incorporation and sustained delivery of the volatile material.

Hence the copolymer and the compatible plasticizer of the polymeric compositions of the present invention can be preferably selected such that the polarity of the plasticized polymeric matrix substantially matches the polarity of the volatile material, wherein the polarities can be evaluated with one of the methods known in the art.

An additional advantage provided by the compositions of the present invention is that they can be formulated as hot melts which have a very low application temperature, typically below 100°C and in some cases if desired can be formulated to have an application temperature below 70°C. This is a particularly desirable property for materials used to incorporate volatile substances as the higher is the processing temperature the bigger is the risk of losing by evaporation significant amounts of the volatile material incorporated during the manufacturing of the composition.

The first essential component of the polymeric composition of the present invention is a copolymer of ethylene with at least another monomer comprising at least a heteroatom.

All copolymers of ethylene with at least another monomer comprising at least a heteroatom are suitable for the present invention.

The term "monomer comprising at least a heteroatom" includes all those monomers which comprise at least a C-X linkage in the molecule wherein X is not C or H. Said C-X linkage is preferably a polar linkage. Preferably the carbon atom is linked to an N, S, F, Cl or O atom. More preferably said polar linkage is part of a carbonyl group and, more preferably, of an ester group. Preferred monomers comprising at least a heteroatom for the present invention are vinyl acetate, vinyl alcohol, methyl acrylate, ethyl acrylate, butyl acrylate, acrylic acid and salts formed therefrom, methacrylic acid and salts formed therefrom, maleic anhydride, glycidyl methacrylate and carbon monoxide.

Suitable copolymers for the present invention can be both block and non-block copolymers, grafted copolymers, copolymers with side chains, or crosslinks and copolymers where ethylene monomers are randomly copolymerized with monomers comprising at least a heteroatom.

Among preferred copolymers of ethylene which are suitable for the present invention are, for example, ethylene-vinyl ester copolymers, ethylene-acrylic ester copolymers, ethylene-methacrylic ester copolymers, ethylene-acrylic acid copolymers and their salts, ethylene-methacrylic acid copolymers and their salts, ethylene-vinyl ester-acrylic acid copolymers, ethylene-vinyl ester-methacrylic acid copolymers, ethylene-vinyl ester-maleic anhydride copolymers, ethylene-acrylic ester-maleic anhydride copolymers, ethylene-vinyl ester-glycidyl methacrylate copolymers, ethylene-acrylic ester-glycidyl methacrylate copolymers, ethylene-maleic anhydride copolymers, ethylene-glycidyl methacrylate copolymers

The monomer comprising at least a heteroatom in the copolymers suitable for the present invention preferably represents from 10% to 90% of the total weight of the copolymer, more preferably at least 14% most preferably at least 18%.

Particularly preferred copolymers for the present invention are ethylene-vinyl acetate copolymers such as those sold under the trade names Elvax™ by Dupont, Evathane™ by Atofina, Escorene™ by Exxon and Levapren™ and Levamelt™ by Bayer and ethylene-acrylic ester copolymers such as those sold under the trade name Lotryl™ by Atofina.

The second essential component in the polymeric compositions of the present invention is a plasticizer or blend of plasticizers comprising at least one heteroatom, which plasticizer or blend of plasticizers is compatible with the copolymer of ethylene with at least another monomer comprising at least a heteroatom. The term "plasticizer comprising at least a heteroatom" includes all those plasticizers which comprise at least a C-X linkage in the molecule wherein X is not C or H. Said C-X linkage is preferably a polar linkage. Preferably the carbon atom is linked to an N, S, F, Cl or O atom. More preferably said polar linkage is part of a carbonyl group and, more preferably, of an ester group.

Suitable plasticizers for use in the polymeric compositions according to the present invention include citric acid esters, low molecular weight polyesters, polyethers, liquid rosin esters, aromatic sulfonamides, phthalates, benzoates, sucrose esters, derivatives of polyfunctional alcohols (where polyfunctional means having 2 or more hydroxyl groups), adipates, tartrates, sebacates, esters of phosphoric acid, fatty acids and diacids, fatty alcohols and diols, epoxidized vegetable oils etc and mixtyres thereof. As already mentioned above, the different polarity of the different compatible plasticisers (measurable with any method known to those skilled in the art, for example water/octanol partition coefficient) can be used to tune the polarity of the polymeric matrix in order to provide a better match with the polarity of the volatile material.

The third essential component of the present invention is a volatile material that is a perfume, which is incorporated and then sustainedly delivered by the compositions of the present invention.

Perfumes are typically composed of many components of different volatility. The present invention, avoiding separation of the components based on their different volatility, allows the sustained delivery of the full perfume bouquet for a long time. In a preferred embodiment of the present invention the volatile material is a perfume which is preferably composed by a plurality of components, more preferably by more than 5 components.

As used herein the term perfume means any odoriferous material. In general, such materials are characterised by a vapour pressure less than the atmospheric pressure at room temperatures. The perfumes employed herein will most often be liquid at room temperatures, but also can be solid such as the various camphoraceous perfumes known in the art. A wide variety of chemicals are known for perfumery uses, including materials such as aldehydes, ketones, esters, alcohols, terpenes and the like. Naturally occurring plant and animal oils and exudates comprising complex mixtures of various chemical components are known for use as perfumes, and such materials can be used herein. The perfumes herein can be relatively simple in their composition or can comprise highly sophisticated, complex mixtures of natural and synthetic chemical components, all chosen to provide any desired odor.

Typical perfumes which can be used in the present invention comprise, for example, woody/earthy bases containing exotic materials such as sandalwood oil, civet, patchouli oil and the like. Other suitable perfumes are for example light, floral fragrances, e.g., rose extract, violet extract and the like. Perfumes can be formulated to provide desirable fruity odours, e.g., lime, lemon, orange and the like.

In short, any chemically compatible material which emanates a pleasant or otherwise desirable odour can be used as a perfume in the present invention.

Perfume materials are described more fully in S. Arctander, Perfume Flavors and Chemicals. Vols. I and II. Aurthor, Montclair, N.J., and the Merck Index, 8th Edition, Merck & Co., Inc. Rahway, N.J.

The volatile material of the present invention is introduced in the polymeric composition in a form which allows the chemicals which constitute said volatile material to be chemically dissolved in the plasticized polymeric matrix. In particular encapsulated volatile materials and chemicals which comprise volatile species covalently bonded to a non volatile one (e.g. pro-perfumes), are not recommended and preferably excluded for use herein as volatile materials according to the present invention. Without being bound to any theory, it is believed that the advantageous properties of the polymeric compositions of the present invention can be seen when the volatile material is solubilized in the plasticized polymeric matrix, as the volatile material release is linked to molecular level interaction between the volatile material and the plasticized polymer matrix. Therefore systems such as encapsulation, which prevent the volatile material from mixing at molecular level with the polymeric matrix, are not preferred for use as volatile materials in the present invention, and are preferably excluded.

The polymeric composition of the present invention comprises from 5% to 75%, more preferably from 10% to 50% by weight of the polymeric composition, of the copolymer of ethylene with at least another monomer comprising at least a heteroatom; from 15% to 60%, preferably from 15% to 40% by weight of the polymeric composition, of the compatible plasticizer or blend of plasticizers comprising at least one heteroatom, and more than 30% of a volatile material; the volatile material is preferably comprised up to a maximum percentage of 90% by weight of the polymeric composition.

The polymeric compositions of the present invention may in addition comprise additional optional components to further improve the processability of the compositions and also the mechanical characteristics as well as other characteristics as tackiness, resistance to ageing by light, oxygen and heat, visual appearance etc., of the objects formed from such polymeric compositions.

Such optional components may include other copolymers that can be included in the formulations to improve their properties for example to increase adhesion or compatibility with substrates. To this purpose preferred optional copolymers are copolymers of styrene and at least one other vinyl or acrylic monomer, copolymers of poly(vinyl alcohol), polyamides, polyether amide copolymers, polyester amide copolymers, polyesters, polyether ester copolymers, polyurethanes, polyethers, poly(2-ethyl-2-oxazoline), copolymers of poly(vinyl pyrrolidone), polyacrylates, copolymers of polyvinyl ethers), etc.

The polymeric compositions of the present invention preferably are thermoplastic polymeric compositions. These can be manufactured by using any known process for manufacturing thermoplastic polymeric compositions and will typically comprise the steps of melting the polymer and then homogeneously blending the plasticizer and the volatile material to form a homogeneous mass that is then cooled to obtain the polymeric composition according to the present invention. Among thermoplastic compositions preferred are those which have low melt temperature and viscosity and therefore are processable as hot melts. In these systems the loss of volatile material upon blending, as well as upon subsequent application in the molten state is minimized.

Other optional components which can be preferably used when the polymeric composition according to the present invention is a thermoplastic composition and preferably has a hot melt rheology are tackifying resins such as rosin derivatives, aliphatic resins, aromatic resins or mixed aliphatic-aromatic resins in order to further increase the adhesion capacity of the compositions of the present invention. The composition can be then formulated in order to be more similar to a true hot melt adhesive, in addition to the capability of releasing volatile materials. Further optional ingredients such as other polymers or copolymers, fillers, crosslinkers, pigments, dyes, antioxidants and other stabilizers, etc can also be added to provide desired properties to the composition.

The polymeric compositions of the present invention may also be prepared using a polymer solution, either as an intermediate or final step. Preparations of this type are well known to those skilled in the art and typically will comprise the steps of dissolving the selected polymer, plasticiser and volatile material in an effective solvent, and heating if necessary to prepare a solution or a gel. The solvent can then be eliminated by evaporation.

Alternatively, the polymeric compositions of the present invention can be prepared in the form of an aqueous emulsion or dispersion.

The techniques for obtaining aqueous emulsions or dispersions of polymers are well known to the skilled man. For example, the selected polymer, plasticiser and volatile material can be blended together as a thermoplastic material. The resulting melt can then be dispersed in water, preferably at a temperature above its melting point, by mixing. Surfactant and/or stabilizing systems known to those skilled in the art can be employed to stabilize the resultant emulsion or dispersion.

Alternatively, a preformed aqueous polymeric dispersion or emulsion can be blended with the selected plasticiser and volatile material. This can be done by adding the ingredients directly to the polymeric dispersion or emulsion, or e.g. by forming an aqueous dispersion of the perfume and plasticiser and blending this with the polymeric dispersion or emulsion. Both procedures result in the formation of an aqueous dispersion of a polymeric composition according to the present invention. Water can be then eliminated by evaporation.

Alternatively, the copolymer can be directly formed in a water dispersion in the presence of the plasticiser and/or of the volatile material. This process can involve the solution or dispersion of monomers or prepolymers in water containing the dispersed volatile material and/or plasticiser. The polymerization can then be initiated to form the polymeric dispersion. If required, the volatile material or plasticiser can be alternatively added subsequently to produce a dispersed polymeric composition according to the present invention.

The polymeric compositions of the present invention due to their rheology and to their adhesion properties are particularly useful to be applied in the molten state onto a selected substrate, and directly adhered thereto. For example they can be applied to the inner surface of a container in a suitable position in order to suitably modify the headspace in the closed container by releasing the volatile material, for example a perfume in order to create a perfumed headspace. Such application can be easily achieved during the manufacturing of the container. In this embodiment, the polymeric composition of the present invention is applied in a conventional hot melt delivery system. Theses systems typically include a melting unit, which maintains the hot melt at the temperature required to have a processable viscosity. The melting unit typically contains a pumping system capable of pumping the hot melt through a hose until it reaches the glue gun, or nozzle. The nozzle can have different geometries according to the desired application form of the glue (coatings, stripes, beads etc). In a typical embodiment, a slot nozzle can be used as the glue gun.

Polymeric compositions according to the present invention may have different applications whenever the release of a volatile material is desired. For example they can be used in air-freshening devices (room-fresheners, car fresheners, toilet rim-blocks etc.), perfumed headspace delivery in packages such as bottles, boxes, bags, etc., cleaning/drying systems (tumble dryers, dishwashers, dry cleaning systems etc.), laundry detergents, fabric conditioners, home care products, personal care products (deodorants, anti-perspirants, shampoos, conditioners, cosmetics, skin moisturizers, makeups etc.), fine fragrances, scented coatings, films, laminates, hygienic articles (fem-care pads, panty liners, diapers, shoe insoles, etc.), scented inks, scented three dimensional objects, disinfectants delivery, insecticides delivery, insect repellants delivery, flavor delivery etc.

### The compositions of the present invention will be illustrated with the following examples:

### Examples

### Example 1

24.75 parts of Elvax® 250, a poly(ethylene-co-vinyl acetate) with a vinyl acetate content of 28 wt% and a melt flow index of 25 dg/min (ASTM D1238), available from Dupont, 9.75 parts of Escorene™ Ultra MV 02528, a poly(ethylene-co-vinyl acetate) with a vinyl acetate content of 27.5 wt% and a melt viscosity at 190 °C of 3100 cps (ExxonMobil method), available from ExxonMobil Chemical, 15 parts of ForalynTM 5020F, a rosin ester plasticiser available from Eastman Chemical and 0.5 parts of IrganoxTM B225, an antioxidant available from Ciba Geigy (Switzerland) were added to a sigma blade mixer and heated to a temperature of about 10-20 °C above the melting point of the polymer (about 120 °C). The ingredients were mixed until a homogeneous mass was obtained. The temperature was then reduced to a point where the mixture was still molten, typically to about 10-20 °C above the melting point of the mixture (about 80 °C in the present case). 50 parts of benzyl acetate, a perfume material available from Sigma Aldrich, were added to the plasticised polymer mixture. The ingredients were mixed until a homogeneous mixture was obtained, and the resultant material was then removed from the mixer, formed as a perfuming block and cooled to room temperature.

### Example 2

24.75 parts of Elvax® 250, a poly(ethylene-co-vinyl acetate) with a vinyl acetate content of 28 wt% and a melt flow index of 25 dg/min (ASTM D1238), available from Dupont, 9.75 parts of Escorene™ Ultra MV 02528, a poly(ethylene-co-vinyl acetate) with a vinyl acetate content of 27.5 wt% and a melt viscosity at 190 °C of 3100 cps (ExxonMobil method), available from ExxonMobil Chemical, 15 parts of ForalynTM 5020F, a rosin ester plasticiser available from Eastman Chemical and 0.5 parts of Irganox™ B225, an antioxidant available from Ciba Geigy (Switzerland) were added to a sigma blade mixer and heated to a temperature of about 10-20 °C above the melting point of the polymers (about 120 °C). The ingredients were mixed until a homogeneous mass was obtained. The temperature was then reduced to a point where the mixture was still molten, typically to about 10-20 °C above the melting point of the mixture (about 80 °C in the present case). 50 parts of eugenol, a perfume material available from Sigma Aldrich was added to the plasticised polymer mixture. The ingredients were mixed until a homogeneous mixture was obtained, and the resultant material was then removed from the mixer, formed as a perfuming block and cooled to room temperature.

### Example 3

39.5 parts of Escorene™ Ultra MV 02528, a poly(ethylene-co-vinyl acetate) with a vinyl acetate content of 27.5% and a melt viscosity at 190 °C of 3100 cps (ExxonMobil method), available from ExxonMobil Chemical, 30 parts of ForalynTM 5020F, a rosin ester plasticiser available from Eastman Chemical and 0.5 parts of IrganoxTM B225, an antioxidant available from Ciba Geigy (Switzerland) were added to a sigma blade mixer and heated to a temperature of about 10-20°C above the melting point of the polymer (about 80 °C). The ingredients were mixed until a homogeneous mass was obtained. The temperature was then reduced to a point where the mixture was still molten, typically to about 10-20 °C above the melting point of the mixture (about 60 °C in the present case). 30 parts of benzyl acetate, a perfume material available from Sigma Aldrich, and this blend was added to the plasticised polymer mixture. The ingredients were mixed until a homogeneous mixture was obtained, and the resultant material was then removed from the mixer, formed as a perfuming block and cooled to room temperature.

### Example 4

39.5 parts of Escorene™ Ultra MV 02528, a poly(ethylene-co-vinyl acetate) with a vinyl acetate content of 27.5% and a melt viscosity at 190 °C of 3100 cps (ExxonMobil method), available from ExxonMobil Chemical, 30 parts of ForalynTM 5020F, a rosin ester plasticiser available from Eastman Chemical and 0.5 parts of IrganoxTM B225, an antioxidant available from Ciba Geigy (Switzerland) were added to a sigma blade mixer and heated to a temperature of about 10-20 °C above the melting point of the polymer (about 80 °C). The ingredients were mixed until a homogeneous mass was obtained. The temperature was then reduced to a point where the mixture was still molten, typically to about 10-20 °C above the melting point of the mixture (about 60 °C in the present case). 30 parts of eugenol, a perfume material available from Sigma Aldrich, and this blend was added to the plasticised polymer mixture. The ingredients were mixed until a homogeneous mixture was obtained, and the resultant material was then removed from the mixer, formed as a perfuming block and cooled to room temperature.

The polymeric compositions of the examples can be easily processed an applied as hot melts, as it is evident from the respective low melting points (between about 60 and about 80 °C).

## Claims

1. A hot melt adhesive polymeric composition comprising:
a) from 5% to 75% by weight of the polymeric composition of a copolymer of ethylene with at least another monomer comprising at least a heteroatom, wherein said monomer comprising at least a heteroatom comprises at least a C-X linkage where X is not C or H and where said linkage is polar;
b) from 15 to 60% by weight of the polymeric composition of a compatible plasticizer or blend of plasticizers comprising at least a heteroatom, and
c) more than 30% by weight of the polymeric composition of a volatile material, wherein the volatile material is a perfume,
wherein the copolymer and the compatible plasticizer or blend of plasticizers comprise a plasticized polymeric matrix, and
wherein the volatile material is chemically dissolved in said plasticized polymeric matrix.

2. A polymeric composition according to claim 1, wherein the monomer comprising at least a heteroatom comprises a carbonyl group.

3. A polymeric composition according to claim 2, wherein the monomer comprising at least a heteroatom comprises an ester group.

4. A polymeric composition according to any preceding claim, wherein the compatible plasticizer or blend of plasticizers comprises a carbonyl group.

5. A polymeric composition according to any of claim 4, wherein the compatible plasticizer or blend of plasticizers comprises an ester group.

6. A polymeric composition according to any preceding claim, wherein the copolymer is an ethylene-vinylacetate copolymer.

7. A polymeric composition according to any preceding claim, wherein the copolymer is from 10% to 50% by weight of the polymeric composition, the compatible plasticizer or blend of plasticizers is from 15% to 40% by weight of the polymeric composition and the volatile material is more than 30% of the total weight of the composition.

8. A polymeric composition according to claim 1, wherein the perfume comprises an aldehyde, a ketone, an alcohol, a terpene or an ester.

9. A polymeric composition according to any preceding claim, wherein the compatible plasticizer is selected form the group consisting of citric acid esters, low molecular weight polyesters, polyethers, rosin esters, aromatic sulfonamides, phthalates, benzoates, sucrose esters, derivatives of polyfunctional alcohols, adipates, tartrates, sebacates, esters of phosphoric acid, fatty acids and diacids, fatty alcohols and diols, epoxidised vegetable oils, and mixtures thereof.

10. A thermoplastic polymeric composition as defined in claim 1.

11. A thermoplastic, melt-processable composition as defined in claim 1.

12. A process for the manufacturing of a closed container comprising the steps of applying a polymeric composition according to any of the preceding claim onto a portion of said container's internal surface.

## Patentansprüche

1. Heißschmelzkleber-Polymerzusammensetzung, umfassend:
a) zu 5 Gew.-% bis 75 Gew.-% der Polymerzusammensetzung ein Copolymer von Ethylen mit mindestens einem weiteren Monomer, das mindestens ein Heteroatom umfasst, wobei das Monomer, das mindestens ein Heteroatom umfasst, mindestens eine C-X-Bindung umfasst, wobei X kein C oder H ist und wobei die Bindung polar ist,
b) zu 15 bis 60 Gew.-% der Polymerzusammensetzung einen kompatiblen Weichmacher oder eine Mischung aus Weichmachern, die mindestens ein Heteroatom umfassen, und
c) zu über 30 Gew.-% der Polymerzusammensetzung ein flüchtiges Material, wobei das flüchtige Material ein Duftstoff ist,
wobei das Copolymer und der kompatible Weichmacher oder die Mischung aus Weichmachern eine weichmacherhaltige Polymermatrix umfassen, und
wobei das flüchtige Material in der weichmacherhaltigen Polymermatrix chemisch gelöst ist.

2. Polymerzusammensetzung nach Anspruch 1, bei der das Monomer, das mindestens ein Heteroatom umfasst, eine Carbonylgruppe umfasst.

3. Polymerzusammensetzung nach Anspruch 2, bei der das Monomer, das mindestens ein Heteroatom umfasst, eine Estergruppe umfasst.

4. Polymerzusammensetzung nach einem der vorstehenden Ansprüche, bei der der kompatible Weichmacher oder die Mischung aus Weichmachern eine Carbonylgruppe umfasst.

5. Polymerzusammensetzung nach einem des Anspruchs 4, bei der der kompatible Weichmacher oder die Mischung aus Weichmachern eine Estergruppe umfasst.

6. Polymerzusammensetzung nach einem der vorhergehenden Ansprüche, bei der das Copolymer ein Ethylen-Vinylacetat-Copolymer ist.

7. Polymerzusammensetzung nach einem der vorstehenden Ansprüche, bei der das Copolymer zwischen 10 Gew.-% und 50 Gew.-% der Polymerzusammensetzung ausmacht, der kompatible Weichmacher oder die Mischung aus Weichmachern zwischen 15 Gew.-% und 40 Gew.-% der Polymerzusammensetzung ausmacht und das flüchtige Material über 30 % des Gesamtgewichts der Zusammensetzung ausmacht.

8. Polymerzusammensetzung nach Anspruch 1, bei der der Duftstoff ein Aldehyd, ein Keton, einen Alkohol, ein Terpen oder einen Ester umfasst.

9. Polymerzusammensetzung nach einem der vorstehenden Ansprüche, bei der der kompatible Weichmacher ausgewählt ist aus der Gruppe bestehend aus Citronensäureestern, niedermolekularen Polyestern, Polyethern, Kolophoniumestern, aromatischen Sulfonamiden, Phthalaten, Benzoaten, Saccharoseestern, Derivaten von polyfunktionellen Alkoholen, Adipaten, Tartraten, Sebacaten, Estern von Phosphorsäure, Fettsäuren und Disäuren, Fettalkoholen und Diolen, epoxidierten Pflanzenölen und Mischungen davon.

10. Thermoplastische Polymerzusammensetzung nach Anspruch 1.

11. Thermoplastische, durch Schmelzen verarbeitbare Polymerzusammensetzung nach Anspruch 1.

12. Prozess zum Herstellen eines geschlossenen Behälters, der die Schritte des Aufbringens einer Polymerzusammensetzung nach einem der vorhergehenden Ansprüche auf einen Teil der Innenfläche des Behälters umfasst.

## Revendications

1. Composition polymère adhésive thermofusible comprenant :
a) de 5 % à 75 % en poids de la composition polymère d'un copolymère d'éthylène avec au moins un autre monomère comprenant au moins un hétéroatome, dans laquelle ledit monomère comprenant au moins un hétéroatome comprend au moins une liaison C-X où X n'est pas C ou H et où ladite liaison est polaire ;
b) de 15 à 60 % en poids de la composition polymère d'un plastifiant compatible ou mélange de plastifiants comprenant au moins un hétéroatome, et
c) plus de 30 % en poids de la composition polymère d'un matériau volatil, dans laquelle le matériau volatil est un parfum,
dans laquelle le copolymère et le plastifiant compatible ou mélange de plastifiants comprennent une matrice polymère plastifiée, et
dans laquelle le matériau volatil est chimiquement dissous dans ladite matrice polymère plastifiée.

2. Composition polymère selon la revendication 1, dans laquelle le monomère comprenant au moins un hétéroatome comprend un groupe carbonyle.

3. Composition polymère selon la revendication 2, dans laquelle le monomère comprenant au moins un hétéroatome comprend un groupe ester.

4. Composition polymère selon l'une quelconque des revendications précédentes, dans laquelle le plastifiant compatible ou mélange de plastifiants comprend un groupe carbonyle.

5. Composition polymère selon l'une quelconque des revendications 4, dans laquelle le plastifiant compatible ou mélange de plastifiants comprend un groupe ester.

6. Composition polymère selon l'une quelconque des revendications précédentes, dans laquelle le copolymère est un copolymère éthylène-vinylacétate.

7. Composition polymère selon l'une quelconque des revendications précédentes, dans laquelle le copolymère va de 10 % à 50 % en poids de la composition polymère, le plastifiant compatible ou mélange de plastifiants va de 15 % à 40 % en poids de la composition polymère et le matériau volatil représente plus de 30 % du poids total de la composition.

8. Composition polymère selon la revendication 1, dans laquelle le parfum comprend un aldéhyde, une cétone, un alcool, un terpène ou un ester.

9. Composition polymère selon l'une quelconque des revendications précédentes, dans laquelle le plastifiant compatible est choisi dans le groupe constitué d'esters d'acide citrique, de polyesters à bas poids moléculaire, de polyéthers, d'esters de rosine, de sulfonamides aromatiques, de phtalates, de benzoates, d'esters de saccharose, de dérivés d'alcools polyfonctionnels, d'adipates, de tartrates, de sébaçates, d'esters d'acide phosphorique, d'acides gras et de diacides, d'alcools gras et de diols, d'huiles végétales époxydées, et leurs mélanges.

10. Composition polymère thermoplastique comme définie dans la revendication 1.

11. Composition thermoplastique, pouvant être traitée en fusion, comme définie dans la revendication 1.

12. Procédé pour la fabrication d'un récipient fermé comprenant les étapes consistant à appliquer une composition polymère selon l'une quelconque des revendications précédentes sur une partie de la surface interne dudit récipient.
